# EUROPÄISCHE PATENTSCHRIFT

(11) **EP 0 379 740 B1**
(45) Veröffentlichungstag und Bekanntmachung des Hinweises auf die Patenterteilung: **01.12.1993**
(21) Anmeldenummer: 89124163.0
(22) Anmeldetag: 29.12.1989
(51) Int. Cl.: A61F 2/18

(54) **Gehörknöchelprothese**
Auditory ossicle prosthesis
Prothèse d'osselet de l'ouie

(30) Priorität: 21.01.1989 DE 3901796
(43) Veröffentlichungstag der Anmeldung: 01.08.1990
(73) Patentinhaber: Kurz, Heinz, D-72144 Dusslingen (DE)
(72) Erfinder: Kurz, Heinz, D-72144 Dusslingen (DE)
(74) Vertreter: Möbus, Rudolf, Dipl.-Ing.

(56) Entgegenhaltungen:
- DE-A- 2 458 932
- US-A- 3 196 462
- US-A- 3 838 468
- US-A- 4 624 672
- US-A- 4 655 776

## Beschreibung

Die Erfindung betrifft eine Gehörknöchelprothese als Schwingungsübertrager mit einer Fläche zur Anlage gegen das Trommelfell, von welcher ein in einer Verdickung endender Stiel ausgeht.

Es ist beispielsweise aus der DE-A-2 458 932 eine derartige Gehörknöchelprothese bekannt, die aus Keramik gefertigt ist. Sie hat den Nachteil, daß sie relativ voluminös ist und teilweise zu Hautreizungen und Entzündungen im Einsatzbereich führt, also nicht für den Körper jedes Patienten verträglich ist. Außerdem besteht die genannte Prothese aus einer Säule, an deren einem Ende eine poröse Platte angeordnet ist, die in Kontakt mit dem Trommelfell kommt. Die Säule ist starr und damit in ihrer Länge während der Operation nicht mehr veränderbar.

In der US-A-3 838 468 wird zwar eine Prothese mit Membran beschrieben, die auch aus Gold bestehen kann, um eine gute Körperverträglichkeit zu erzielen, doch dient diese Prothese nicht dem Ersatz der Gehörknöchelchenkette und weist ebenfalls keine Möglichkeit zur Anpassung ihrer Länge während der Implantation auf.

Der Erfindung liegt die Aufgabe zugrunde, eine Gehörknöchelprothese zu schaffen, die eine gute Körperverträglichkeit aufweist und die sich während der operativen Implantation in ihrer Länge den anatomischen Gegebenheiten anpassen läßt.

Die gestellte Aufgabe wird bei einer Gehörknöchelprothese mit den eingangs genannten Merkmalen erfindungsgemäß durch die Merkmale des kennzeichnenden Teils des Hauptanspruchs gelöst. Sie kann auch ganz oder teilweise aus Titan gefertigt sein.

Erfindungsgemäß ausgebildete Gehörknöchelprothesen lassen sich sehr dünn gestalten und bestehen aus einem Material, das sich auch auf anderen Gebieten als gut körperverträglich erwiesen hat. Durch die Ausbildung eines Krümmungsbereiches im Stiel läßt sich während des operativen Einsetzens der Prothese die Gesamtlänge des Stieles den individuellen Gegebenheiten anpassen.

Die Anlagefläche der Gehörknöchelprothese kann an einer Feingoldscheibe ausgebildet sein, wobei durch noppenförmige Vorsprünge einerseits der Anlagedruck am Trommelfell auf mehrere Bereiche verteilt wird und andererseits eine nach allen Seiten gleich gut wirksame Absicherung gegen ein Abgleiten der Prothese von der Innenseite des Trommelfells erreicht wird. Eine gute Haftung der Anlagefläche an einer Schleimhaut läßt sich auch durch ein Aufrauhen der Anlagefläche erreichen.

Bei einer bevorzugten Ausführungsform ist die Gehörknöchelprothese einstückig aus einem Feingolddraht gefertigt, wobei die Anlagefläche durch eine aus dem Feingolddraht gewundene Spirale oder Windung einer Wendel dieses Drahtes gebildet ist und eine gekrümmte oder eckige Begrenzungslinie haben kann. Diese bevorzugte Ausführungsform ist nicht nur fertigungstechnisch günstig. Bei ihr erfolgt auch eine nur linienförmige Anlage der Prothese am Trommelfell, jedoch über eine große Linienlänge. Dementsprechend werden nur schmale Trommelfellbereiche durch die Prothese abgedeckt und dadurch eine Entzündungsgefahr beseitigt. Bestehende Längendifferenzen des Prothesenstieles lassen sich hier ohne zusätzliche Krümmungen des Feingolddrahtes im Stielbereich allein aus der Drahtspirale oder Drahtwendel kompensieren oder in der Spirale oder Wendel unterbringen. In jedem Falle läßt sich die Feingold-Gehörknöchelprothese mit gratfreien Rändern ausbilden, wobei bei einer Prothese mit Spirale oder Wendel das dortige Drahtende gratfrei an der Spirale oder Wendel angeschweißt sein kann, beispielsweise durch Laser-Schweißung.

Nachfolgend werden zwei Ausführungsbeispiele einer Gehörknöchelprothese anhand der beiliegenden Zeichnung näher erläutert.

Im einzelnen zeigen:
- Fig. 1: eine Seitenansicht einer Gehörknöchelprothese;
- Fig. 2: eine Draufsicht auf die Anlagefläche der Gehörknöchelprothese in Richtung des Pfeiles II in Fig. 1;
- Fig. 3: eine Seitenansicht einer zweiten Ausführungsform einer Gehörknöchelprothese;
- Fig. 4: eine Draufsicht auf die Gehörknöchelprothese in Richtung des Pfeiles IV in Fig. 3;
- Fig. 5: eine Teilseitenansicht, teilweise im Schnitt, von einer abgewandelten Prothese nach Fig. 3 und 4;
- Fig. 6: eine der Fig. 4 entsprechende Draufsicht auf eine eckig ausgebildete Prothesenwendel.

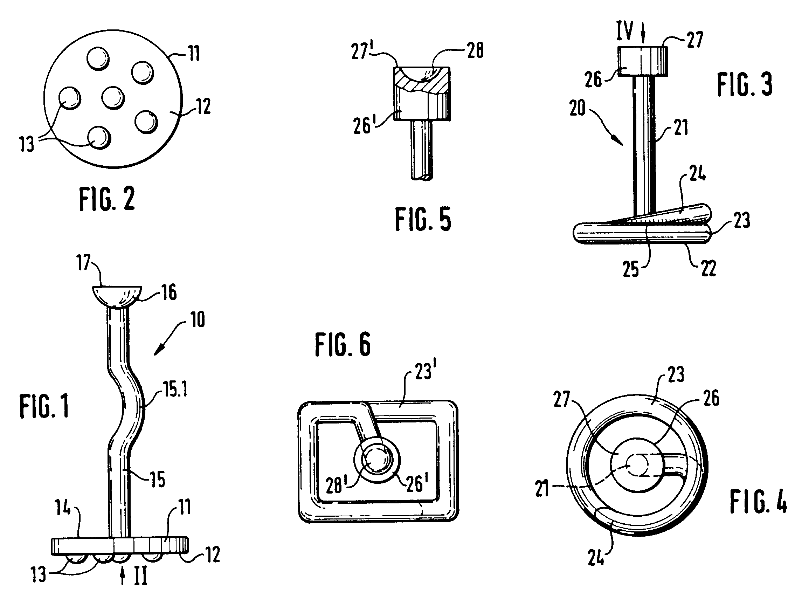

Die in den Fig. 1 und 2 dargestellte Gehörknöchelprothese 10 besteht aus einer Feingoldscheibe 11, die mit ihrer einen Seite eine Anlagefläche 12 bildet, die gegen das Trommelfell eines Patienten zur Anlage kommt. Diese Anlagefläche 12 ist mit gleichmäßig verteilten runden Noppen 13 versehen. In der Mitte der Rückseite 14 der Feingoldscheibe 11 ist ein aus einem Feingolddraht gefertigter Stiel 15 mittig angesetzt, an dessen freiem Ende eine Verdickung 16 in Form einer Kugelkalotte ausgebildet ist. Die Kugelkalotte 16 bildet eine ebene kreisförmige Kontaktfläche 17. In seiner Mitte weist der Stiel 15 einen gekrümmten Bereich 15.1 auf, der eine Längenreserve bildet und eine Veränderung des gegenseitigen Abstandes von Feingoldscheibe 11 und ebenfalls aus Feingold bestehender Kugelkalotte 16 erlaubt.

Die Fig. 3 und 4 zeigen eine Gehörknöchelprothese 20, die einstückig aus einem Feingolddraht gefertigt ist. Die Grundform ist dem Ausführungsbeispiel nach Fig. 1 und 2 ähnlich, doch ist der Stiel 21 der Prothese über seine ganze Länge geradlinig ausgebildet. Die Anlagefläche 22 der Gehörknöchelprothese 20 ist durch die Windung einer Wendel 23 gebildet, zu welcher der Feingolddraht gebogen wird. Der Endbereich 24 des Feingolddrahtes ist zu einer zweiten Windung der Wendel angesetzt, schräg geschliffen und mit der Rückseite der die Anlagefläche 22 bildenden Windung der Wendel 23 mit Laserhilfe fest verschweißt, wie in Fig. 3 durch eine Schweißnaht 25 angedeutet ist. Am freien Ende des Stieles 21 ist der Feingolddraht zu einem Walzenkörper 26 verdickt, der eine ebene Kontaktfläche 27 wie beim Ausführungsbeispiel nach Fig. 1 und 2 bildet.

Anstelle einer Drahtwendel 23 kann auch eine Drahtspirale ausgebildet sein. Die von ihr gebildete Anlagefläche 22 kann zur besseren Haftung aufgerauht sein. Die Drahtwendel 23 muß nicht die dargestellte Kreisform haben, sondern kann auch eine ovale oder gemäß Fig. 6 eine eckige Anlagefläche begrenzen. Eine Längenreserve für den Stiel 21 ist hier in der Drahtwendel 23 oder der Drahtspirale gegeben, so daß auch bei dieser Ausführungsform der Gehörknöchelprothese bei der Operation der Abstand zwischen Kontaktfläche 22 und Anlagefläche 27 individuell eingestellt werden kann. In Fig. 6 sind gleiche Teile mit den gleichen Bezugsziffern, gegänzt durch einen Beistrich, bezeichnet.

Fig. 5 zeigt das Walzenkörperende 26' einer Gehörknöchel-Teilprothese. Dort ist in der Kontaktfläche 27' eine Vertiefung 28 ausgebildet, in welche der Steigbügel eintauchen kann.

## Patentansprüche

1. Gehörknöchelprothese als Schwingungsübertrager, mit einer Fläche (12) zur Anlage gegen das Trommelfell, von welcher ein in einer Verdickung (16) endender Stiel (15) ausgeht, dadurch gekennzeichnet, daß sie aus Feingold gefertigt ist und mindestens ihr Stiel (15, 21) aus einem Golddraht besteht, der zur Veränderung des gegenseitigen Abstandes von Anlagefläche (12) und verdicktem Stielende (16) einen Krümmungsbereich (15.1) aufweist.

2. Gehörknöchelprothese nach Anspruch 1 oder 2, dadurch gekennzeichnet, daß das verdickte Stielende (26) walzenartig ausgebildet ist.

3. Gehörknöchelprothese nach einem der Ansprüche 1 bis 3, dadurch gekennzeichnet, daß ihr verdicktes Stielende (16, 26) eine ebene Kontaktfläche (17, 27) aufweist.

4. Gehörknöchelprothese nach einem der Ansprüche 1 bis 3, dadurch gekennzeichnet, daß ihr verdicktes Stielende (26') auf seiner Kontaktseite eine Vertiefung (28) aufweist.

5. Gehörknöchelprothese nach einem der Ansprüche 1 bis 5, dadurch gekennzeichnet, daß ihre Anlagefläche (12) an einer Feingoldscheibe (11) ausgebildet und mit noppenförmigen Vorsprüngen (13) versehen ist.

6. Gehörknöchelprothese nach einem der Ansprüche 1 bis 5, dadurch gekennzeichnet, daß sie einstückig aus einem Feingolddraht gefertigt ist und daß die Anlagefläche (22) durch eine aus dem Feingolddraht eckig oder rund gewundene Spirale oder Wendel (23) gebildet ist.

7. Gehörknöchelprothese nach Anspruch 7, dadurch gekennzeichnet, daß eines der Drahtenden (24) an der Spirale oder Wendel (23) gratfrei angeschweißt ist.

8. Gehörknöchelprothese nach einem der Ansprüche 1 bis 8, dadurch gekennzeichnet, daß sie auf ihrer Anlagefläche (12, 22) aufgerauht ist.

9. Gehörknöchelprothese nach einem der Ansprüche 1 bis 9, dadurch gekennzeichnet, daß sie anstelle aus Feingold mindestens teilweise aus Titan gefertigt ist.

## Claims

1. Auditory ossicle prosthesis as oscillatory transmitter, comprising a surface (12) for abutting the tympanic membrane, from which extends a stem (15) with a thicker end, **characterised in that** it is made of fine gold and that at least its stem (15, 21) is made of a gold wire which has curved areas (15.1) for the purpose of altering the distance between abutment surface (12) and thickened end of the stem (16).

2. Auditory ossicle prosthesis according to claim 1 or 2, **characterised in that** the thickened end of the stem (26) is arranged like a cylinder.

3. Auditory ossicle prosthesis according to claims 1 to 3, **characterized in that** the thickened end of its stem (16, 26) comprises a plane contact surface (17, 27).

4. Auditory ossicle prosthesis according to one of claims 1 to 3, **characterised in that** the thickened end of its stem (26') has a recess (28) on its contact side.

5. Auditory ossicle prosthesis according to one of claims 1 to 5, **characterised in that** its abutment surface (12) is arranged on a disc of fine gold (11) and is provided with burlshaped protrusions (13).

6. Auditory ossicle prosthesis according to one of claims 1 to 5, **characterised in that** it is made in one piece of fine gold wire and that the abutment surface (22) is formed by a cornered or round wound spiral or coil (23) of fine gold wire.

7. Auditory ossicle prosthesis according to claim 7, **characterised in that** one of the wire ends (24) is welded without edge to the spiral or coil (23).

8. Auditory ossicle prosthesis according to one of claims 1 to 8, **characterised in that** its abutment surface is roughened.

9. Auditory ossicle prosthesis according to one of claims 1 to 9, **characterised in that** it is at least partially made of titanium instead of fine gold.

## Revendications

1. Prothèse d'osselet de l'ouïe comme transmetteur de vibrations avec une surface (12) pour appui contre le tympan, de laquelle sort un montant (15) se terminant en un renflement (16), caractérisée en ce qu'elle est fabriquée en or fin et en ce que son montant (15, 21) au moins est constitué d'un fil en or qui présente une zone de courbure (15.1). pour la modification de la distance réciproque de la surface d'appui (12) et de l'extrémité du montant renflée (16).

2. Prothèse d'osselet de l'ouïe selon la revendication 1 ou 2, caractérisée en ce que l'extrémité du montant renflée (26) est formée en forme de cylindre.

3. Prothèse d'osselet de l'ouïe selon l'une des revendications 1 à 3, caractérisée en ce que son extrémité de montant renflée (16, 26) présente une surface de contact plane (17, 27).

4. Prothèse d'osselet de l'ouïe selon l'une des revendications 1 à 3, caractérisée en ce que son extrémité de montant renflée (26') présente sur son côté de contact un renfoncement (28).

5. Prothèse d'osselet de l'ouïe selon l'une des revendications 1 à 5, caractérisée en ce que sa surface d'appui (12) est formée sur une rondelle d'or fin (11) et est munie de saillies en forme de nopes (13).

6. Prothèse d'osselet de l'ouïe selon l'une des revendications 1 à 5, caractérisée en ce qu'elle est fabriquée d'une pièce à partir d'un fil en or fin et en ce que la surface d'appui (22) est formée par une spirale ou hélice (23) fléchie en coin ou en rond à partir du fil en or fin.

7. Prothèse d'osselet de l'ouïe selon la revendication 7, caractérisée en ce qu'une des extrémités du fil (24) est soudée sans ébarbure à la spirale ou hélice (23).

8. Prothèse d'osselet de l'ouïe selon l'une des revendications 1 à 8, caractérisée en ce qu'elle est rendue rugueuse sur sa surface d'appui (12, 22).

9. Prothèse d'osselet de l'ouïe selon l'une des revendications 1 à 9, caractérisée en ce qu'elle est fabriquée au moins partiellement en titane au lieu d'or fin.
